# EUROPEAN PATENT SPECIFICATION

(11) **EP 1 752 433 B1**
(45) Date of publication and mention of the grant of the patent: **03.10.2012**
(21) Application number: 05017340.0
(22) Date of filing: 10.08.2005
(51) Int. Cl.: C07C 2/08, C07C 7/00, C07C 11/02

(54) **Improved drying process for linear alpha-olefins**
Verbessertes Trocknungsverfahren linearer Alpha-Olefine
Procédé de sechage amélioré d'alpha-oléfines

(43) Date of publication of application: 14.02.2007
(73) Proprietor: Linde AG, 80331 München (DE); Saudi Basic Industries Corporation, Riyadh 11422 (SA)
(72) Inventor: Bölt, Heinz, 82515 Wolfratshausen (DE); Fritz, Peter, Dr., 82008 Unterchaching (DE); Mosa, Fuad, 11422 Riyadh (SA); Ali, Talal, Dr., 11422 Riyadh (SA)
(74) Representative: Winkler, Andreas Fritz Ernst

(56) References cited:
- EP-A- 0 706 983
- DE-A1- 1 568 461
- DE-A1- 19 807 226
- DE-C1- 4 338 414
- DE-C1- 4 338 415

## Description

The present invention relates to a method for preparing linear alpha-olefins (LAO).

Processes for the oligomerisation of ethylene utilizing a homogeneous catalyst are widely known. For example DE 43 38 414 C1 discloses a process for the oligomerisation of ethylene to obtain linear alpha-olefins, wherein ethylene is catalytically converted in an empty tubular reactor utilizing a catalyst comprising a zirconium component and an aluminum component. The process is advantageously carried out in a continuous mode wherein gaseous and liquid outlet streams are obtained. The liquid outlet stream usually contains solvent, catalyst, dissolved ethylene and linear alpha-olefins. To avoid plugging and reactor fouling the catalyst contained in the liquid outlet stream has to be immediately deactivated to avoid further oligomerisation.

According to the prior art, the obtained liquid outlet stream may be treated with water, alcohol or fatty acids to deactivate the catalytic components. Upon deactivation with water, large quantities of liquid linear alpha-olefins saturated with water are obtained, requiring the removal of the water for further processing. So far, water was removed by passing the liquid outlet stream through an adsorber, comprising for example molecular sieve, alumina or silica, to adsorb water and catalytic components thereon.

However, due to the large quantities of liquid linear alpha-olefins saturated with water obtained, excessively large adsorber vessels and thus high investment costs are required. Further, for regeneration of such large adsorbers, huge amounts of regeneration gas is required which also would result in high costs for the disposal thereof.

EP 0 706 983 discloses the preparation of linear alpha-olefins by oligomerisation of ethylene. After the reaction, the catalyst is deactivated and separated from the product by using a chelate resin. The reaction mixture is washed with water and separated into oligomer and solvent by distillation. Separation of water from the oligomer by distillation is not disclosed.

DE 198 07 226 A1 discloses a process for deactivation of complex metal organic catalysts in homogeneous processes, such as the oligomerisation of ethylene, wherein the obtained product solution is mixed with metal hydroxide in a protonic solvent, wherein the activation and isolation of the catalysts from the organic phase are carried out in one step. In this process the amount of the aqueous phase is kept relatively low, just enough to ensure the deactivation of the catalyst. This small amount of aqueous phase would be dissolved or entrained in the organic phase, but is too small for the formation of a separate aqueous phase.

It is therefore an object of the present invention to provide a method for preparing linear alpha-olefins which overcomes the drawbacks of the prior art, especially to provide a method comprising an improved drying step of the linear alpha-olefins obtained resulting in reduced costs, and reduced plant wastes.

This object is achieved by a method for preparing linear alpha-olefins by oligomerisation of ethylene, comprising the steps of:
(i) oligomerising ethylene in a reactor in the presence of a solvent and a catalyst;
(ii) transferring a liquid organic outlet stream of the reactor, containing solvent, catalyst, dissolved ethylene and linear alpha-olefins, to a catalyst deactivation section;
(iii) deactivating the catalyst by washing the outlet stream with an aqueous basic phase to obtain a deactivated catalyst containing aqueous phase and a water saturated organic phase;
(iv) separating the aqueous phase and the organic phase from step (iii);
(v) transferring the water saturated organic phase to a distillation column;
(vi) distilling the water saturated organic phase; and
(vii) separating the distilled organic and aqueous phases.

In a preferred embodiment the separation in step (iv) and/or (vii) takes place in a phase separator.

Also, after step (vii) the organic phase may be passed through an adsorber.

In addition, it is also preferred that the adsorber is zeolite, molecular sieve, alumina, silica, or mixtures thereof.

Preferably, the aqueous basic phase contains alkali metal hydroxide, preferably NaOH and/or KOH, NH₃, organic amines or mixtures thereof.

Moreover, after deactivating the catalyst with an aqueous basic phase in step (ii) the obtained organic phase may be additionally washed with water.

In one aspect the aqueous phases obtained in step (iv) and/or (vii) are recycled.

Surprisingly, it was found that utilizing the inventive method, only small or no adsorbers at all are required for removing the residual water contained in the organic stream of linear alpha-olefins. This is achieved by distilling the water saturated organic phase to remove the major amounts of water. After distillation, the organic phase may then, if necessary at all, passed through an adsorber. As only small or no adsorbers are required, reduced investments costs result. Further, only small amounts of regeneration gas are needed to regenerate the adsorber. Further, the inventive method results in a minimization of plant wastes, for example molecular sieve of the adsorber, regeneration offgas. Finally, the amount of consumed process water is reduced, since the water may be recovered for re-use. In the inventive method, larger quantities of aqueous phase are employed which allow a phase separation of an organic and an aqueous phase since this large quantities of aqueous phase cannot be dissolved in the organic phase. It is within the skill of an artisan to adjust the amount of aqueous solvent to obtain a phase separation.

Additional features and advantages of the invention method will now become apparent from the detailed description of the preferred embodiment thereof.

Ethylene is oligomerised in a suitable reactor, for example an empty tubular reactor, as disclosed in DE 43 38 414 C1, utilizing a catalyst comprising a zirconium component and an aluminum component. A suitable zirconium component is zirconium tetraisobutyrate, and a suitable aluminum component is ethyl aluminum sesquichloride.

The oligomerisation is carried out under conditions well known in the art. From the reactor, a liquid organic outlet stream is obtained, containing solvent (for example toluene), catalyst, ethylene dissolved in the solvent, and linear alpha-olefins. This liquid organic outlet stream is transferred to a catalyst deactivation section. To avoid plugging and fouling of the reactor and the pipes connecting the reactor and the catalyst deactivation section, it is preferred to deactivate the catalyst after exit from the reactor as soon as possible. According to the present invention, the catalyst is deactivated by washing the obtained organic outlet stream with an aqueous basic phase, for example water and sodium hydroxide. The organic phase and the aqueous basic phase may be separated, and, preferably, the obtained organic phase may be additionally washed with water, followed by a separation of the washed organic phase and the water.

The water saturated organic phase is then transferred to a distillation column. The distillation column may be designed to easily allow separation of the water contained in that organic phase. Especially, attention has to be drawn to avoid potential foaming and formation of hydrates. Foaming in distillation columns may be avoided by selection of appropriate operating conditions (pressure, temperature) and proper design of tray type and column geometry. Further, the formation of hydrates may be avoided by proper selection of the process conditions (e.g. pressure, temperature, tube wall temperature in heat exchangers, refrigerant temperature). However, the respective process design is well known for someone skilled in the art. After distillation, the water content of the bottoms product of the distillation column is significantly reduced due to the higher volatility of water compared to higher linear alpha-olefins forming substantially the bottoms product.

Most of the water contained in the water saturated organic phase is routed to the column overhead of the distillation column and may be condensed in its condenser together with light linear alpha-olefins. Thus, the water may be recovered in liquid state and may be separated from light linear alpha-olefins in a phase separator.

Since most of the water is recovered in the liquid phase, the residual water content in the linear alpha-olefins is significantly reduced.

To achieve required moisture levels of the final LAO products, if necessary, the organic phases may be passed through adsorbers. However, these adsorbers may be small compared to the prior art and thus reduce investments costs are necessary and also reduced amounts of regeneration gas to regenerate the adsorbers.

The features disclosed in the foregoing description and in the claims may, both separately and in any combination thereof, be material for realizing the invention in diverse forms thereof.

## Claims

1. Method for preparing linear alpha-olefins by oligomerisation of ethylene, comprising the steps of:
(i) oligomerising ethylene in a reactor in the presence of a solvent and a catalyst;
(ii) transferring a liquid organic outlet stream of the reactor, containing solvent, catalyst, dissolved ethylene and linear alpha-olefins, to a catalyst deactivation section;
(iii) deactivating the catalyst by washing the outlet stream with an aqueous basic phase to obtain a deactivated catalyst containing aqueous phase and a water saturated organic phase;
(iv) separating the aqueous phase and the organic phase from step (iii);
(v) transferring the water saturated organic phase to a distillation column;
(vi) distilling the water saturated organic phase; and
(vii) separating the distilled organic and aqueous phases.

2. Method according to claim 1, wherein the separation in step (iv) and/or (vii) takes place in a phase separator.

3. Method according to claim 1 or 2, wherein after step (vii) the organic phase is passed through an adsorber.

4. Method according to claim 3, wherein the adsorber is zeolite, molecular sieve, alumina, silica, or mixtures thereof.

5. Method according to any of the preceding claims, wherein the aqueous basic phase contains alkali metal hydroxide, preferably NaOH and/or KOH, NH₃, organic amines or mixtures thereof.

6. Method according to any of the preceding claims, wherein after deactivating the catalyst with an aqueous basic phase in step (ii) the obtained organic phase is additionally washed with water.

7. Method according to any of the preceding claims, wherein the aqueous phases obtained in step (iv) and/or (vii) are recycled.

## Patentansprüche

1. Verfahren zum Herstellen linearer alpha-Olefine durch Oligomerisation von Ethylen, welches die Schritte umfasst:
(i) Oligomerisieren von Ethylen in einem Reactor in der Gegenwart eines Lösungsmittels und eines Katalysators;
(ii) Überführen eines flüssigen organischen Auslassstromes, der Lösungsmittel, Katalysator, gelöstes Ethylen und lineare alpha-olefine enthält, von dem Reaktor zu einem Katalysatordeaktivierungsabschnitt;
(iii) Desaktivieren des Katalysators durch Waschen des Auslassstromes mit einer wässrigen basischen Phase, um eine deaktivierten Katalysator enthaltende wässrige Phase und eine wassergesättigte organische Phase zu erhalten;
(iv) Abtrennen der wässrigen Phase und der organischen Phase aus Schritt (iii);
(v) Überführen der wassergesättigten organischen Phase zu einer Destillationssäule;
(vi) Destillieren der wassergesättigten organischen Phase; und
(vii) Abtrennen der destillierten organischen und wässrigen Phasen.

2. Verfahren nach Anspruch 1, wobei die Abtrennung in Schritt (iv) und/oder (vii) in einem Phasenseparator stattfindet.

3. Verfahren, nach Anspruch 1 oder 2, wobei nach Schritt (vii) die organische Phase durch einen Adsorber geführt wird.

4. Verfahren nach Anspruch 3, wobei der Adsorber Zeolith, Molekularsieb, Aluminiumoxid, Siliziumoxid oder Mischungen derselben ist.

5. Verfahren nach einem der vorangehenden Ansprüche, wobei die wässrige basische Phase Alkalimetallhydroxid, bevorzugt NaOH und/oder KOH, NH₃, organische Amine oder Mischungen derselben enthält.

6. Verfahren nach einem der vorangehenden Ansprüche, wobei nach Deaktivieren des Katalysators mit einer wässrigen basischen Phase in Schritt (ii) die erhaltene organische Phase zusätzlich mit Wasser gewaschen wird.

7. Verfahren nach einem der vorangehenden Ansprüche, wobei die wässrigen Phasen, die in Schritt (iv) und/oder (vii) erhalten werden, recycliert werden.

## Revendications

1. Procédé de préparation d'alpha-oléfines linéaires par oligomérisation de l'éthylène, comprenant les étapes qui consistent :
(i) à oligomériser l'éthylène dans un réacteur en présence d'un solvant et d'un catalyseur ;
(ii) à transférer un flux de sortie organique liquide du réacteur, contenant le solvant, le catalyseur, l'éthylène dissous et des alpha-oléfines linéaires, à une section de désactivation de catalyseur ;
(iii) à désactiver le catalyseur en lavant le flux de sortie avec une phase aqueuse basique pour obtenir un catalyseur désactivé contenant une phase aqueuse et une phase organique saturée en eau ;
(iv) à séparer la phase aqueuse et la phase organique de l'étape (iii) ;
(v) à transférer la phase organique saturée en eau à une colonne de distillation ;
(vi) à distiller la phase organique saturée en eau ;
et
(vii) à séparer les phases organique et aqueuse distillées.

2. Procédé selon la revendication 1, dans lequel la séparation dans l'étape (iv) et/ou (vii) a lieu dans un séparateur de phases.

3. Procédé selon la revendication 1 ou 2, dans lequel après l'étape (vii) la phase organique est passée à travers un adsorbeur.

4. Procédé selon la revendication 3, dans lequel l'adsorbeur est une zéolite, un tamis moléculaire, une alumine, une silice, ou leurs mélanges.

5. Procédé selon l'une des revendications précédentes, dans lequel la phase aqueuse basique continent de l'hydroxyde de métal alcalin, de préférence du NaOH et/ou du KOH, de NH₃, des amines organiques ou leurs mélanges.

6. Procédé selon l'une des revendications précédentes, dans lequel après désactivation du catalyseur avec une phase aqueuse basique dans l'étape (ii) la phase organique obtenue est en outre lavée avec de l'eau.

7. Procédé selon l'une des revendications précédentes, dans lequel les phases aqueuses obtenues dans l'étape (iv) et/ou (vii) sont recyclées.
